(19) Europäisches Patentamt
European Patent Office
Office européen des brevets

(11) **EP 2 037 952 B1**

(12) **EUROPEAN PATENT SPECIFICATION**

(45) Date of publication and mention
of the grant of the patent:
**21.10.2015 Bulletin 2015/43**

(51) Int Cl.:
***A61K 38/18*** *(2006.01)*     ***A61P 27/02*** *(2006.01)*

(21) Application number: **07720997.1**

(22) Date of filing: **28.04.2007**

(86) International application number:
**PCT/CN2007/001424**

(87) International publication number:
**WO 2007/128222 (15.11.2007 Gazette 2007/46)**

(54) **VITREOUS ADMINISTRATION OF ERYTHROPOIETIN**

GLASKÖRPERVERABREICHUNG VON ERYTHROPOETIN

ADMINISTRATION D'ÉRYTHROPOÏÉTINE PAR INJECTION DANS LE VITRÉ

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **29.04.2006 CN 200610026242**

(43) Date of publication of application:
**25.03.2009 Bulletin 2009/13**

(73) Proprietors:
- **Shanghai Institutes for Biological Sciences, Chinese Academy of Sciences**
  **Shanghai 200031 (CN)**
- **Philadelphia Health and Education Corporation D/B/A**
  **Philadelphia, PA 19102 (US)**
- **Vimetrics LLC**
  **Pennsylvannia 19063 (US)**

(72) Inventors:
- **XU, Guotong**
  **Shanghai 200031 (CN)**
- **LI, Weiye**
  **Shanghai 200031 (CN)**
- **SINCLAIR, Stephen, H.**
  **Media, PA 19063 (US)**

(74) Representative: **Osha Liang**
**2, rue de la Paix**
**75002 Paris (FR)**

(56) References cited:
**WO-A1-00/61164**     **WO-A2-03/103608**
**CN-A- 1 607 957**

- **TSAI JAMES C ET AL: "Intravitreal administration of erythropoietin and preservation of retinal ganglion cells in an experimental rat model of glaucoma." CURRENT EYE RESEARCH NOV 2005 LNKD- PUBMED:16282136, vol. 30, no. 11, November 2005 (2005-11), pages 1025-1031, XP008123253 ISSN: 0271-3683**
- **DATABASE BIOSIS [Online] BIOSCIENCES INFORMATION SERVICE, PHILADELPHIA, PA, US; April 2004 (2004-04), WU L ET AL: "Effects of intraocular administration of erythropoietin on intraocular pressure and neuronal apoptosis of retinal tissues in experimental glaucomatous rat eyes" XP002586612 Database accession no. PREV200510302160 & IOVS, vol. 45, no. Suppl. 1, April 2004 (2004-04), page U790, ANNUAL MEETING OF THE ASSOCIATION-FOR-RESEARCH-IN-VISION-AND-OPH THALM OLOGY; FT LAUDERDALE, FL, USA; APRIL 24 -29, 2004 ISSN: 0146-0404**
- **DATABASE BIOSIS [Online] BIOSCIENCES INFORMATION SERVICE, PHILADELPHIA, PA, US; 2005, WU L ET AL: "Immunoreactivity of erythropoietin and its receptor in retina and optic nerve in an experimental rat model of glaucoma" XP002586613 Database accession no. PREV200600053172 & IOVS, vol. 46, no. Suppl. S, 2005, page 1316, ANNUAL MEETING OF THE ASSOCIATION-FOR-RESEARCH-IN-VISION-AND-OPH THALM OLOGY; FT LAUDERDALE, FL, USA; MAY 01 -05, 2005 ISSN: 0146-0404**

**(Cont. next page)**

EP 2 037 952 B1

- **ZHANG JINGFA ET AL: "Intravitreal injection of erythropoietin protects both retinal vascular and neuronal cells in early diabetes." INVESTIGATIVE OPHTHALMOLOGY & VISUAL SCIENCE FEB 2008 LNKD- PUBMED:18235022, vol. 49, no. 2, February 2008 (2008-02), pages 732-742, XP002587023 ISSN: 0146-0404**
- **WEISBAUPT J.H. ET AL.: 'Effect of Erythropoietin Axotomy-Induced Apoptosis in Rat Retinal Ganglion Cells' INVESTIGATIVE OPHTHALMOLOGY & VISUAL SCIENCE vol. 45, no. 5, 2004, pages 1514 - 1522, XP008102788**
- **Kai Jaquet ET AL: "Erythropoietin and VEGF Exhibit Equal Angiogenic Potential", Microvascular Research, vol. 64, no. 2, 1 September 2002 (2002-09-01), pages 326-333, XP055149437, ISSN: 0026-2862, DOI: 10.1006/mvre.2002.2426**
- **Donald S. Fong ET AL: "Retinopathy in Diabetes", Diabetes Care, 1 January 2004 (2004-01-01), pages s84-87, XP055149442, United States DOI: 10.2337/diacare.27.2007.S84 Retrieved from the Internet: URL:http://care.diabetesjournals.org/conte nt/27/suppl_1/s84.extract**

Remarks:
The file contains technical information submitted after the application was filed and not included in this specification

**Description**

**FIELD OF THE INVENTION**

The present invention relates to the use of erythropoietin for the manufacture of a medicament for the treatment and/or prevention of diabetic retinopathy, the pathogenesis of which is based upon derangement of the blood retinal barrier (BRB) or retinal vascular, glial or neuronal or RPE cell death, as well as to corresponding compositions.

**BACKGROUND**

Diabetic retinopathy (DR) is the leading cause of blindness in persons aged 20- to 70- years. The direct cause of DR remains largely unknown. The notion that DR is solely a microvascular complication in diabetes has been challenged in recent years. Evidence exists that all classes of cells within the retina are involved in a multiplicity of disease processes even in the early stages of diabetes. The early clinical features of DR, such as breakdown of blood-retinal barrier (BRB) and a variety of visual deficits, support this concept in humans. Because of the gradual but accelerating deterioration associated with DR, the treatment of DR should be implemented before DR progresses to irreversible stages. However, at present all treatments are implemented for well established DR at injunctions in which pathology studies demonstrate irreversible retinal vascular and neuronal cell death, and while some treatments may produce limited improvement in visual function or vascular leakage, most have been reported only to forestall further injury.

Fong et al. (Diabetes Care, 1 January 2004, pages s84-87) discloses an overview of treatment of diabetic retinopathy

**SUMMARY OF THE INVENTION**

The present invention relates to the use of erythropoietin for the manufacture of a medicament for the treatment of a retinal disorder associated with breakdown of blood-retinal-barrier (BRB) or a retinal disorder associated with apoptosis of cells of retina, wherein the retinal disorder is diabetic retinopathy. According to one or more embodiments, the medicament may comprise a composition for invitreal or transscleral injection. According to one or more embodiments, the cells of retina are photoreceptor cells of the outer nuclear layer, neurons of the inner nuclear layer, retinal pigment epithelial cells, and/or choriocapillaris endothelial cells. The EPO used in the injectable composition may include recombinant human erythropoietin. The composition may include that amount of erythropoietin effective to substantially inhibit breakdown of the blood-retinal-barrier and/or substantially inhibit apoptosis of at least one of retinal vascular, glial, or neuronal cells, or retinal pigment epithelial cells, or choriocapillaris epithelial cells in a mammal's eye. According to one or more embodiments of the invention, the composition may include an amount of EPO effective to provide the vitreous of a mammalian eye with an EPO concentration of about 0.33 ng/$\mu$l to about 13.33 ng/$\mu$l based on the vitreous volume of the human eye. According to one or more embodiments, the composition may comprise about 1.33 $\mu$g to about 60 $\mu$g of EPO per eye or about 0.23 to about 9.2 units per eye (e.g., 2.3 units per eye).

The present invention also relates to a composition comprising erythropoietin for use in the treatment of a retinal disorder associated with breakdown of blood-retinal-barrier or apoptosis of cells of retina, wherein the retinal disorder is diabetic retinopathy. According to one or more embodiments, the cells of retina are photoreceptor cells of the outer nuclear layer, neurons of the inner nuclear layer, retinal pigment epithelial cells, and/or choriocapillaris endothelial cells. According to one or more embodiments, the composition may comprise an effective amount of erythropoietin of about 1.33 $\mu$g to about 60 $\mu$g per eye. According to one or more embodiments, the composition may comprise an effective amount of erythropoietin of about 0.23 to about 9.2 units per eye. According to one or more embodiments, the composition may comprise human recombinant erythropoietin.

**BRIEF DESCRIPTION OF THE DRAWINGS**

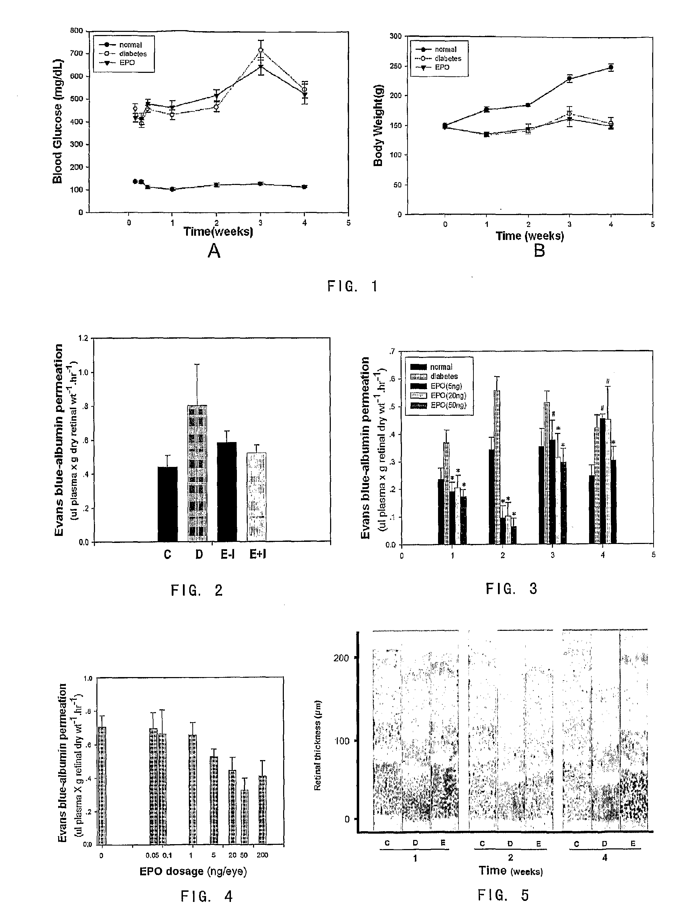

FIG. 1(A-B) are plots illustrating blood glucose levels (A) and body weights (B) of diabetic rats with and without intravitreal EPO treatment at 4-week period after onset of diabetes. Each data point was expressed as mean$\pm$S.E. with n $\geq$ 7. ($\bullet$) non-diabetic control rats; ($\bigcirc$) Diabetic rats without EPO treatment; ($\blacktriangledown$) Diabetic rats treated with a single intravitreal EPO injection (50 ng/eye) at the day of diabetes onset. Sham injections (normal saline) were performed in both control and diabetic rats.

FIG. 2 is a graph illustrating the effect of insulin and EPO on Evans blue-albumin permeation into the retina in STZ-diabetic rats at 2-weeks after onset of diabetes: C, non-diabetic control; D, diabetic rats treated without insulin; E-I, diabetic rats treated with EPO but without Insulin injection; E+I, diabetic rats treated with both Insulin and EPO.

The EPO was injected into the vitreous (5 ng/eye) for both EPO treated groups. Sham injection (normal saline) was performed to both control and diabetes groups. In the E+I group, Insulin was given at a low dosage (4 IU per week) by subcutaneous injection, so that the blood glucose level could be maintained at high level. There is no significant difference between the rats treated with Insulin and those without Insulin treatment (n=6, p>0.05).

FIG. 3 is a graph illustrating the time course of Evans blue-albumin permeation (EBP) into the retina of STZ-diabetic rats treated with different doses of EPO. Three dosages of EPO (5, 20 and 50 ng) were tested. Sham injection (normal saline) was performed to both normal and diabetes groups. Each data point was expressed as mean±S.E. The sample numbers of each group were equal to or more than 7. All the groups with * are significantly different from the diabetes group (p<0.05), and the three groups with # are not significantly different in comparison with diabetes group (p>0.05).

FIG. 4 is a graph illustrating dose-dependent protection by EPO on BRB of STZ-diabetic rats. Different doses of EPO (0.05 to 200ng) were injected into vitreous at the onset of diabetes. Sham injection (normal saline) was performed in both the control and diabetic rats. The rats were sacrificed after two weeks of the treatments. Each point represents mean±S.E. from 6 rats.

FIG. 5 is a graph illustrating retinal thickness in STZ-diabetic rats by intravitreal injection of EPO. The dose of EPO was 50 ng/eye. Sham injection (normal saline) was performed to both non-diabetic control rats and diabetic rats. Three rats were sacrificed at each week up to 4 weeks; representative sections are shown here. C: non-diabetic control; D: diabetes; E: EPO-treated diabetic rats. The thickness of the retina was measured on H/E stain samples under the magnification of 200 X.

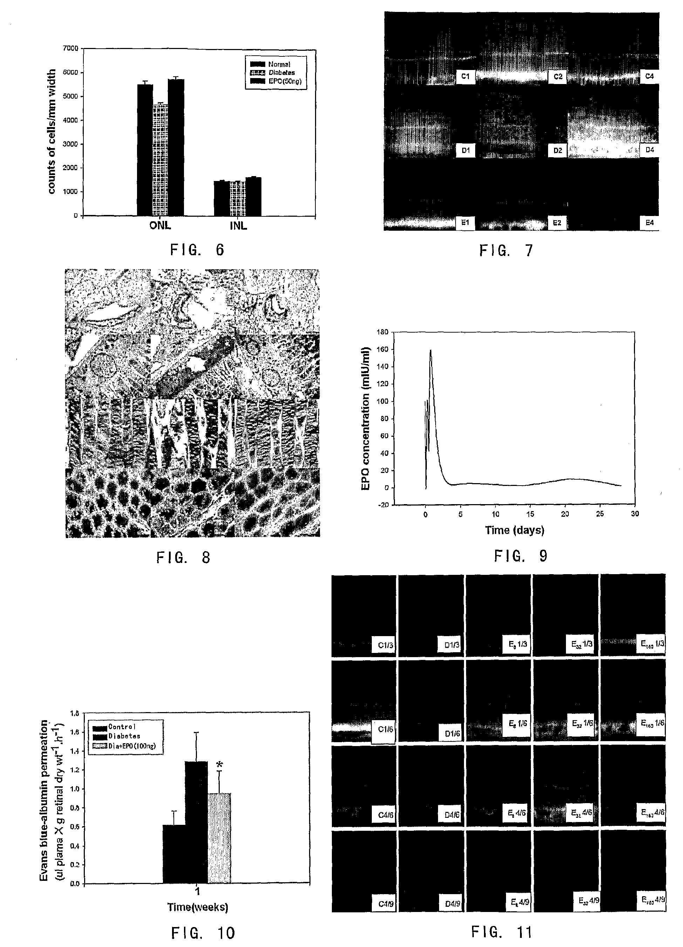

FIG. 6 is graph illustrating prevention loss of retinal neurons, glial cells, Mueller, and vascular endothelial cells in STZ-diabetic rats by EPO. The dose of EPO was 50 ng/eye for the EPO group. The diabetes and non-diabetic control received the Sham injection (normal saline). Three rats from each group were sacrificed at 4 weeks. The number of retinal neurons in different layers was counted systematically at the same orientation from 12 sections of each rat. Briefly, 12 sections from each rat (6 from each eye) were examined; Cell nuclei in 25um in width were counted. Therefore, each datum point represents mean±S.E. of 72 counts for each group. The cell nuclei after H/E staining under the magnification of 1,000X were counted to represent cell number. C: non-diabetic control; D: diabetes; E: EPO-treated diabetic rats. The cell count in ONL of diabetic rat retina was significantly reduced (n=12, p<0.001) and resumed completely by EPO treatment (n=12, p<0.001). For INL, the cell count in EPO treated rats was significantly higher than that of diabetic rats (n=12, p<0.001).

FIG. 7 shows representative micrographs of EPO protection of retinal neurons from death detected by TUNEL staining. The diabetic rats were treated with a single intravitreal injection of EPO (E) with the dose of 50 ng/eye. Sham injection (normal saline) was performed in both non-diabetic control rats (C) and diabetic rats (D). The number 'n' behind the letters indicates the weeks after onset of diabetes, i.e., Cn: control rats of n weeks; Dn: diabetic rats of n weeks; En: EPO-treated diabetic rats of n weeks.

FIG. 8 shows representative electron micrographs of different retinal cells of controls and diabetic rats that were treated or untreated with intravitreal injection of EPO. C: non-diabetic control; D: diabetic rats; E: diabetic rats treated with EPO (50 ng/eye). The rats were sacrificed one week after the onset of diabetes. The figures show the changes in retinal vascular endothelial cells (en-C, en-D and en-E, 9,700X), retinal pigment epithelial cells (rpe-C, rpe-D and rpe-E, 5800X), rod and cone outer segment layer cells (r/c-C, r/c-D and r/c-E, 9,700X), and the outer nuclear layer cells (ONL, onl-C, onl-D and onl-E, 5800X). The changes resulting from diabetes are indicted by the arrows, and include apoptotic vascular endothelial cell, edema/exudates around the vascular endothelial cells (en-D), rupture of Bruch's membrane (BM), and collagen accumulation in the widened space between RPE and BM (rpe-D), the destroyed structure and inter-cell space left by the dissolved discs(r/c-D), and the evident pyknosis in ONL (onl-D). The changes were largely prevented in the EPO treated rats.

FIG. 9 is a plot illustrating the elimination half-life of vitreous EPO after intravitreal injection into normal rats. EPO in the vitreous was detected by Elisa. Each data point represents an average of duplicated measurements of two samples.

FIG 10 illustrates Evans blue-albumin permeation into the retina of diabetic rats treated with EPO or normal saline. *p<0.05, n =6.

FIG. 11 illustrates detection of apoptosis of retinal cells by TUNEL staining in STZ treated rats. C: control; D: diabetic; E: EPO-treated. The numbers of n/m: the intravitreal injection was performed at n weeks and samples were harvested at m weeks after diabetes onset. $E_x$: the dose of EPO is x mU/eye.

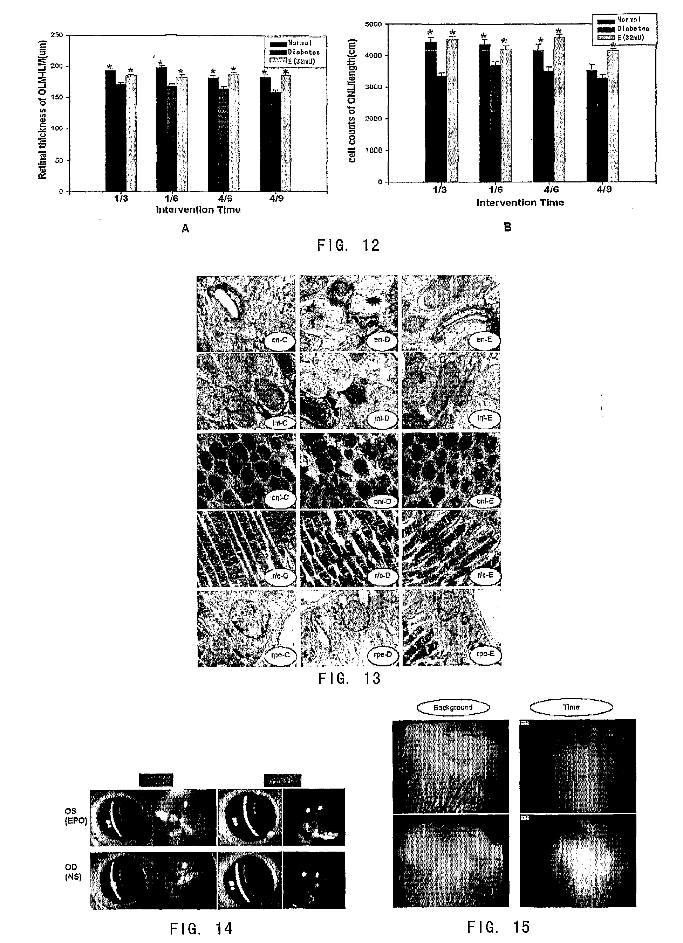

FIG. 12 illustrates retinal thickness and ONL cell counts in STZ rats treated with or without EPO (32mU/eye). C: Normal control; D: Diabetes; E: Diabetic rats treated with EPO (32 mU/eye). * p<0.05 when compared with diabetic groups.

FIG. 13 illustrates the retinal morphological characters observed under EM. EPO was given 1 week after the onset of diabetes and the rats were killed a week afterward. en: endothelial cells (9,700x); inl: inner nuclear layer (5,800 x); onl: outer nuclear layer (5,800x); r/c: rod and cone layer (9,700x); rpe: retinal pigment epithelium (5,800x). The changes in the diabetic retina were marked with different symbols. EPO administration significantly ameliorated these changes.

FIG. 14 shows photos of slit lamp and funduscopy of the rabbits at 2 weeks and 1 month after EPO injection. There is no difference can be found between EPO-injected eyes and NS-injected eyes. No sign for inflammation, cataract or retinopathy was found.

FIG. 15 illustrates FFA examination in rabbits at 2 weeks and 1 month after intravitreal injection of EPO. There is no significant leakage in the eye fundus. LT: color view; RT, LL and RL showed the color funduscopy at different time after the dye injected.

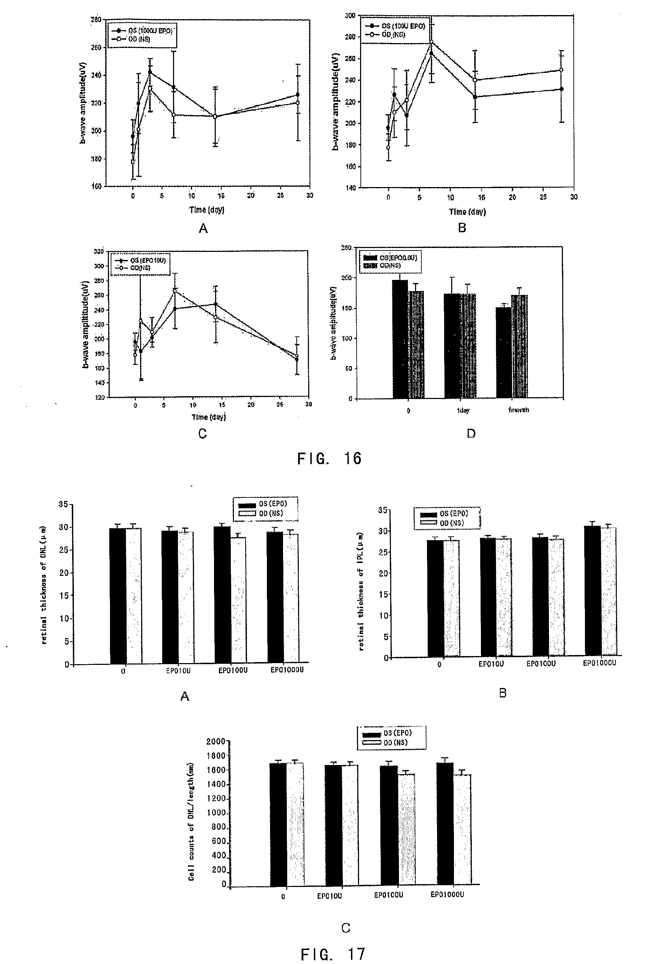

FIG. 16 shows graphs illustrating ERG examination of the rabbits after EPO intravitreal injections. A (1000U/eye); B (100U/eye); C (10U/eye); D (0.6U/eye, repeating injections). There is a peak of b-wave altitude within 1 week after intravitreal injection, then it comes down to normal level. There is no difference between EPO-injected and NS-injected eyes.

FIG. 17 illustrates histological study on the rabbits' retinas after ITV. A: the retinal thickness of ONL; B: the retinal thickness in IPL; C: the cell counts of ONL.

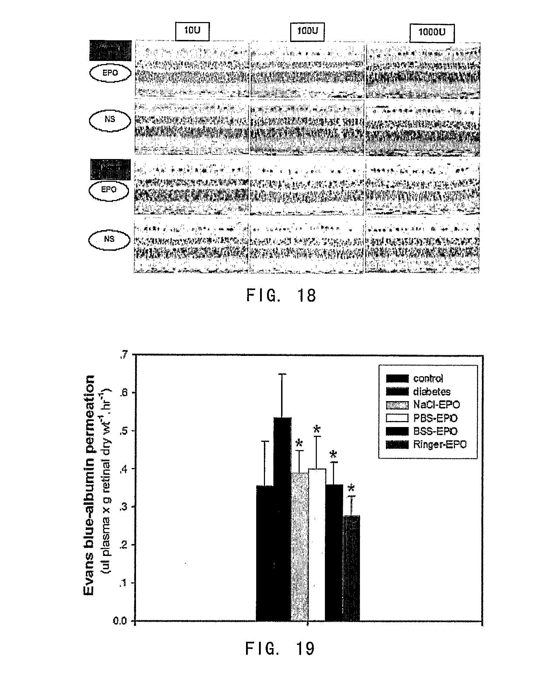

FIG. 18 illustrates Histological observation of the rabbit retina under light microscopy. There is no significant difference between EPO (10U, 100U or 1,000U)-injected eyes and NS-injected eyes. That means the intravitreal injection of EPO is safe for at least 2 month (data for 2 months were not shown, we just completed the experiment).

FIG. 19 is a graph illustrating Evans blue-albumin permeation (EBP) into the retina of STZ-diabetic rats treated with different solvents buffered-EPO. The dose of EPO is 8mU/eye with the injection volume $2\mu l$. Four solvents were tested: NS, PBS, BSS and Ringer. Sham injection (normal saline) was performed to both normal and diabetes groups. Each datum point was expressed as mean$\pm$S.E. The sample numbers of each group were equal to 6. All the groups with * are significantly different from the diabetes group ($p<0.05$).

## DETAILED DESCRIPTION

The present invention relates to the use of erythropoietin (EPO) for the manufacture of a medicament for the treatment of a retinal disorder associated with breakdown of blood-retinal-barrier or a retinal disorder associated with apoptosis of cells of retina, wherein the retinal disorder is diabetic retinopathy. The treatment may refer to the treatment of diabetic retinopathy in a mammal by administration of an erythropoietin (EPO) medicament to the vitreous of the mammal's (e.g., human) eye. The treatment of retinal disorder may include, for example, slowing the rate of vision loss due to a retinal disorder of the mammalian eye. Intravitreal administration or transscleral administration (i.e., across the sclera) of EPO substantially inhibits degradation of the blood-retinal-barrier (BRB) and substantially inhibits or mitigates apoptosis of diseased retinal cells, such as photoreceptor cells of the outer nuclear layer (ONL), retinal pigment epithelial cells (RPE), and choriocapillaris epithelial cells. Therefore, the claimed uses and compositions pertain to treating diabetic retinopathy.

Diabetic retinopathy is a progressive and pervasive disorder of retinal, vascular, and neuronal cells under diabetic condition. The most common cause of vision loss in diabetic retinopathy is macular edema. As the disease progresses with progressive occlusion of normal vessels, neovascularization proliferates and vitreous hemorrhage and retinal detachment ensue.

The EPO that is intravitreally (or transsclerally) administered in accordance with an embodiment of the use of the present invention may have part or all of the primary structural conformation (*i.e.,* continuous sequence of amino acid residues) and one or more of the biological properties (*e.g.,* immunological properties and *in vitro* biological activity) and physical properties (*e.g.,* molecular weight) of the naturally-occurring mammalian EPO. For example, the amino acid sequence of the naturally-occurring EPO may be substantially identical to an amino acid sequence, which corresponds to GenBank Accession No. CAA26095.

Naturally occurring EPO may be purified and isolated. The term "purified and isolated" herein means substantially free of unwanted substances so that the EPO is useful for inhibiting BRB breakdown and retinal cell apoptosis. For example, one may have a recombinant human EPO substantially free of other human proteins or pathological agents.

The EPO that is intravitreally (or transsclerally) administered may also include other proteins or peptides that have the biological activity of human EPO, i.e., inhibiting BRB breakdown and retinal cell apoptosis. These other proteins or peptides may include, for example, EPO analogs, EPO isoforms, EPO mimetics, EPO fragments, hybrid EPO proteins, fusion proteins oligomers and multimers of the above, homologues of the above, including receptor blockers or agonists, glycosylation pattern variants of the above, and mutants of the above, regardless of the method of synthesis or manufacture thereof including but not limited to, recombinant vector expression whether produced from cDNA or genomic DNA, synthetic, transgenic, and gene activated methods.

Specific examples of EPO include highly pure, recombinant EPO (e.g., EPOGEN, Amgen, Thousand Oaks, USA, Epoetin alfa (e.g., EPREX, ERYPO, Janssen-Cilag, Ltd., Sauderton, UK and PROCRIT, Ortho Biotech LP, Bridgewater, NJ), novel erythropoiesis stimulating protein (NESP) (e.g., ARANESP, Amgen, Thousand Oaks, USA), a hyperglycosylated analog of recombinant human EPO described in European patent application EP640619, human EPO analog--human serum albumin fusion proteins described in the international patent application WO9966054, EPO mutants described in international patent application WO9938890, EPO omega, which may be produced from an Apa I restriction fragment of the human EPO gene described in U.S. Pat. No. 5,688,679, altered glycosylated human EPO described in the international patent application WO9911781, PEG conjugated erythropoietin analogs described in WO9805363 or U.S. Pat. No. 5,643,575. Specific examples of cell lines modified for expression of endogenous human erythropoietin are described in international patent applications WO9905268 and WO9412650.

The EPO used in accordance with embodiments of the invention may be modified, for example, to increase the half-life, efficacy, metabolism, and/or potency of the protein. The EPO mimetic may be an EPO conjugate, such as those taught by Burg et al. in U.S. Pat. No. 6,340,742. The EPO mimetic may contain substituted or artificial amino acids, such as those taught by Connolly et al. in U.S. Pat. No. 6,310,078. The EPO mimetic may be a protein or small molecule that has little or no similarity to native EPO, but inhibits BRB breakdown and retinal cell apoptosis. It is thus contemplated that various changes may be made to EPO without appreciable loss of its biological utility or activity (e.g., EPO ability to inhibit BRB breakdown and retinal cell apoptosis).

According to an embodiment of the invention, the EPO may be administered to the vitreous of the eye using any intravitreal or transscleral administration technique. For example, the EPO may be administered to the vitreous of the eye by intravitreal injection of the EPO. Injectables for such use may be prepared in conventional forms, either as a liquid solution or suspension or in a solid form suitable for preparation as a solution or suspension in a liquid prior to injection, or as an emulsion. Carriers may include, for example, water, saline (e.g., normal saline (NS), phosphate-buffered saline (PBS), balanced saline solution (BSS)), sodium lactate Ringer's solution, dextrose, glycerol, ethanol, and the like; and if desired, minor amounts of auxiliary substances, such as wetting or emulsifying agents, buffers, and the like may be added. Proper fluidity may be maintained, for example, by using a coating such as lecithin, by maintaining the required particle size in the case of dispersion and by using surfactants. By way of example, the EPO may be dissolved in a pharmaceutically effective carrier and be injected into the vitreous of the eye with a fine gauge hollow bore needle (e.g., 30 gauge, ½ or 3/8 inch needle) using a temporal approach (e.g., about 3 to about 4 mm posterior to the limbus for human eye to avoid damaging the lens).

One skilled in the art will appreciate that other means for injecting and/or administering EPO to the vitreous of the eye may also be used. These other means may include, for example, intravitreal medical delivery devices disclosed in U.S. Pat. No. 6,251,090, and U.S. Pat. No. 6,299,603. These devices and methods may include, for example, intravitreal medicine delivery devices, such as disclosed in U.S. Patent No. 6,251,090, and biodegradable polymer delivery members that are inserted in the eye for long term delivery of medicaments, such as disclosed in U.S. Patent Publication No. 2005/0250737. These devices and methods may further include transscleral delivery devices, such as disclosed in U.S. Patent Application Publication Nos. 2005/0208103, 2005/0074497, and 2005/0064010 as well as "Transscleral drug delivery for posterior segment disease." Adv Drug Delivery Rev 52: 37-48.

The EPO that is administered to the eye may be provided in a pharmaceutical composition. Such compositions should be stable during manufacture and storage and must be preserved against contamination from microorganisms, such as bacteria and fungi. Various antibacterial and antifungal agents, such as parabens, chlorobutanol, phenol, ascorbic acid, and thimerosal, can control microorganism contamination. Isotonic agents, such as sugars, polyalcohols such as manitol, sorbitol, and sodium chloride may be included in the composition. Agents that delay absorption of the EPO may also be included in the pharmaceutical composition. Such absorption delaying agents, may include, for example, aluminum monostearate and gelatin.

Sterile injectable solutions may be prepared by incorporating the EPO in an appropriate solvent with one or a combination of ingredients, followed by sterilization. Generally, dispersions are prepared by incorporating the EPO into a sterile vehicle that contains a basic dispersion medium, and any other required ingredients. Sterile powders for the preparation of sterile injectable solutions may be used. Methods of preparation include vacuum drying and freeze-drying that yield a powder containing the active ingredient and any desired ingredient from a sterile solution.

It will be appreciated that other devices and methods of intravitreally administering EPO to the eye may also be used. These devices and methods may include, for example, intravitreal medicine delivery devices, such as disclosed in U.S. Patent No. 6,251,090, and biodegradable polymer delivery members that are inserted in the eye for long term delivery of medicaments, such as disclosed in U.S. Patent Publication No. 2005/0250737.

The EPO may be administered to the vitreous of the mammal's eye in a therapeutically effective amount to treat and/or prevent BRB breakdown and/or retinal cell or retinal pigment epithelial cell apoptosis prior to and/or upon detection of diabetic retinopathy. According to an embodiment of the invention, the EPO may be administered to the vitreous of the mammal's eye at the onset and/or upon detection of diabetes and/or the early stages of diabetic retinopathy. Administration of the EPO to the vitreous of the mammal's eye at this time can be substantially effective at inhibiting BRB breakdown as well as retinal cell and retinal pigment epithelial cell apoptosis.

The therapeutically effective amount of EPO may be that amount of EPO that is nontoxic but sufficient to provide the desired effect and performance at a reasonable benefit/risk ratio attending any medical treatment. According to an embodiment of invention, the amount of EPO intravitreally administered to the mammalian eye may be that amount effective to substantially inhibit or mitigate breakdown of the BRB as well as retinal cell and retinal pigment epithelial cell apoptosis (or retinal ischemia) over a predetermined duration of time. The predetermined duration of time may be that duration time in which breakdown of the blood retinal barrier and/or retinal cell or retinal pigment epithelial cell apoptosis is effectively mitigated. This duration of time may be, for example, from about 1 day to about 3 months in duration.

By way example, the amount of EPO intravitreally administered to the mammal's eye may be that amount effective to provide the vitreous of the mammalian eye with an EPO concentration of about 0.33 ng/$\mu$l to about 13.33 ng/$\mu$l based on the vitreous volume of the eye. It has been shown in STZ-diabetic rats that at this concentration, which is provided by a 50 ng to about 200 ng of EPO intravitreal injection, breakdown of the BRB and retinal cell apoptosis can be substantially mitigated for up to about 4 weeks. Thus, for patients exhibiting early signs of diabetic retinopathy (or no retinopathy, per se, but, having demonstrable neuronopathy or BRB breakdown) an amount of EPO intravitreally administered to the mammal's eye effective to provide the vitreous of a mammalian eye with an EPO concentration of about 0.33 ng/$\mu$l to about 13.33 ng/$\mu$l may be administered to the eye of the patient episodically at about 3 week to about 3 month (e.g., about 4 week) increments or longer to maintain the efficacy of the treatment and to substantially inhibit BRB breakdown and retinal cell or retinal pigment epithelial cell apoptosis.

For humans, an amount of EPO effective to and to substantially inhibit BRB breakdown and retinal cell or retinal pigment epithelial cell apoptosis may be about 1.33 $\mu$g to about 60 $\mu$g per eye or about 0.23 units per eye to about 9.2 units per eye (e.g., about 2.3 units per eye). The about 1.33 $\mu$g to about 60 $\mu$g of EPO may be provided in, for example, an about 90 $\mu$l to about 100 $\mu$l solution, which may be intravitreally administered to the human eye. In a further embodiment of the invention, the amount of EPO administered to the human eye may be about 1.33 $\mu$g to about 60 $\mu$g per eye (e.g., about 15 $\mu$g per eye) (or about 0.23 to about 9.2 units per eye) and be provided in an about 90 $\mu$l to about 100 $\mu$l solution.

It will be understood, however, that the specific dose level and frequency of dosage for any particular patient may be varied and depends upon a variety of factors, including the activity of the specific EPO composition employed, the metabolic stability and length of action of the EPO composition, the age, body weight, general health, sex, diet, mode and time of administration, rate of excretion, drug combination, the severity of the particular condition, and the host undergoing therapy.

In accordance with another embodiment of the present invention, the EPO may be administered to the vitreous by forcing the expression of the EPO from a target cell. The EPO that is expressed in the vitreous of the eye may be an expression product of an EPO gene. The sequence of the gene encoding native human EPO, as well as methods of obtaining the same, are described in, e.g., U.S. Pat. Nos. 4,954,437 and 4,703,008, as well as in Jacobs et al. (1985) Nature 313:806-810; Lin et al. (1985) Proc. Natl. Acad. Sci. USA 82:7580; International Publication Number WO 85/02610; and European Patent Publication Number 232,034 B1. In addition, the sequences of the genes encoding native feline, canine and porcine EPO are known and readily available (GenBank Accession Nos.: L10606; L13027; and L10607, respectively), and the sequence of the gene encoding monkey (Macaca mulatta) is also known and available (GenBank Accession No.: L10609).

The expression of EPO in the vitreous may be performed by introducing an agent into target cells that increases expression of EPO. The target cells may include cells within the retina or retinal pigment epithelium or ex vivo cells, such as embryonic stem cells and/or multipotent adult progenitor cells, which are transplanted into the retina following introduction of the agent.

The agent may comprise natural or synthetic EPO nucleic acids that are incorporated into recombinant nucleic acid constructs, typically DNA constructs, capable of introduction into and replication in the cell. Such a construct may include a replication system and sequences that are capable of transcription and translation of a polypeptide-encoding sequence in a given target cell.

Other agents may also be introduced the target cells to promote EPO expression in a target tissue. For example, agents that increase the transcription of a gene encoding EPO; increase the translation of an mRNA encoding EPO, and/ or those that decrease the degradation of an mRNA encoding EPO could be used to increase EPO levels. Increasing the rate of transcription from a gene within a cell may be accomplished by introducing an exogenous promoter upstream of the gene encoding EPO. Enhancer elements, which facilitate expression of a heterologous gene may also be employed.

One method of introducing the agent into a target cell involves using gene therapy. Gene therapy in accordance with embodiments of the use of the present invention may be used to express EPO from a target cell in vivo or in vitro.

An example of a gene therapy method involves using a vector including a nucleotide encoding EPO. Examples of vectors that may be used include, for example, viral vectors (such as adenoviruses ('Ad'), adeno-associated viruses (AAV), and retroviruses), liposomes and other lipid-containing complexes, and other macromolecular complexes capable of mediating delivery of a polynucleotide to a target cell.

The vectors may also comprise other components or functionalities that further modulate gene delivery and/or gene expression, or that otherwise provide beneficial properties to the targeted cells. Such other components include, for example, components that influence binding or targeting to cells (including components that mediate cell-type or tissue-specific binding); components that influence uptake of the vector nucleic acid by the cell; components that influence localization of the polynucleotide within the cell after uptake (such as agents mediating nuclear localization); and components that influence expression of the polynucleotide. Such components may also include markers, such as detectable and/or selectable markers that can be used to detect or select for cells that have taken up and are expressing the nucleic acid delivered by the vector. Such components may be provided as a natural feature of the vector (such as the use of certain viral vectors which have components or functionalities mediating binding and uptake), or vectors may be modified to provide such functionalities.

Vectors for use in expressing EPO in embodiments of the present invention include viral vectors, lipid based vectors, and other vectors that are capable of delivering nucleotide to the target cells. The vector may be a targeted vector, especially a targeted vector that preferentially binds to retinal cells or retinal pigment epithelium. Viral vectors for use in embodiments of the invention may also be those that exhibit low toxicity to a target cell and induce production of therapeutically useful quantities of EPO in a tissue-specific manner.

One example of a viral vector that may be used for expressing EPO in a target cell is adeno-associated virus (AAV). Such AAV vectors that may be used to express EPO in target cells are described in U.S. Pat. No. 6,325,998. Other vectors including viral and non-viral vectors well known in the art and described above may also be used.

In addition to viral vector-based methods, non-viral methods may also be used to introduce an EPO gene into a target cell. An example of a non-viral gene delivery method according to an embodiment of the invention employs plasmid DNA to introduce an EPO nucleic acid into a cell. Plasmid-based gene delivery methods are generally known in the art.

The vectors that encode the expression of EPO may be delivered to the target cell in the form of an injectable preparation containing pharmaceutically acceptable carrier such as saline, for example, as necessary. Other pharmaceutical carriers, formulations and dosages may also be used.

Where the target cell comprises a cell of the retina or retinal pigment epithelium, the vector may be delivered by direct injection, at an amount sufficient for the EPO to be expressed to a degree that allows for highly effective therapy. By injecting the vector directly into the retina, it is possible to target the gene rather effectively, and to minimize loss of the recombinant vectors. This type of injection has been demonstrated in other tissues to allow local transfection of a desired number of cells, as in the case of cardiac myocytes, in the affected myocardium, thereby maximizing therapeutic efficacy of gene transfer, and minimizing the possibility of an inflammatory response to viral proteins.

Where the target cell is a cultured cell (e.g., embryonic stem cell or multipotent adult progenitor cells) that is later transplanted into the vitreous or retina, the vectors may be delivered by direct injection into the culture medium. An EPO nucleic acid transfected into cells may be operably linked to any suitable regulatory sequence, including a specific promoter and enhancer.

The transfected target cells (e.g., embryonic stem cells or multipotent adult progenitor cells) may then be transplanted to the retina by well known transplantation techniques. By first transfecting the target cells in vitro and than transplanting the transfected target cells to the vitreous or retina, the possibility of inflammatory response in the retina is minimized compared to direct injection of the vector into the vitreous or retina.

The EPO used in embodiments of the present invention may be expressed for any suitable length of time including transient expression and stable, long-term expression. In one embodiment, the EPO will be expressed in therapeutic amounts for a suitable and defined length of time so as to treat and/or prevent BRB breakdown and/or retinal cell retinal pigment epithelial cell apoptosis associated with diabetic retinopathy and/or other retinal disorders.

The treatment and/or prevention of diabetic retinopathy, including BRB breakdown and/or retinal cell or retinal pigment epithelial cell apoptosis, may be determined as compared to a control, standard, or baseline. The level of BRB breakdown and/or retinal cell apoptosis may be quantified using methods known in the art. For example, a decrease, as compared with a control, standard, or baseline, indicates successful treatment. Additionally, a decrease in altered vision abnormalities as defined in the art indicates successful treatment. The quantified amounts of BRB breakdown and/or retinal cell or retinal pigment epithelial cell apoptosis may be compared with a suitable control to determine if the levels are decreased. The sample to be tested may be compared with levels for the specific individual from previous time points (e.g., before having diabetes and/or diabetic retinopathy, or during various phases of treatment for the diabetic retinopathy), or with levels in normal individuals or suitable controls.

An individual who is being treated for diabetic effects on the retina may be monitored by determining the levels of BRB breakdown at various time points. Such levels of BRB breakdown may be determined before treatment, during treatment, and after treatment. A decrease in the level of BRB breakdown, as described herein, indicates successful treatment. BRB breakdown may be measured using methods now known or those developed in the future. See Kohner E. M., et al. Diabetic Retinopathy Metabolism, 25:1985-1102 (1975). For example, BRB breakdown may be measured using a fluorescein angiogram or by measuring the vision loss in a patient. Moreover, BRB breakdown may be measured using vision measurement systems, such as CS, MAVES, CVS, multi-focal ERG, HVF, etc.

The following examples are included to describe embodiments of the invention. One skilled in the are will appreciate that the techniques disclosed in the examples which follow represent techniques discovered by the inventor to function well in the practice of the invention, and thus can be considered to constitute preferred modes for its practice.

However, those of skill in the art should, in light of the present disclosure, appreciate that many changes may be made in the specific embodiments that are disclosed and still obtain a like or similar result.

## EXAMPLES

## EXAMPLE 1

In the present study, we used streptozotocin (STZ)-induced diabetic rats as an animal model to study two fundamental questions of the early course of diabetic retinopathy and the effect of EPO: 1) what is the prominent type of cellular injury in the retina after the onset of diabetes? 2) what is the earliest time frame of vascular and non-vascular injury in diabetic retina? Based on the understanding of these two questions, a new therapeutic option was explored by a single dose of EPO injected into the vitreous at the day of diabetes established. The protective functions of EPO on BRB, vascular and neuronal cells have been clearly demonstrated.

## RESEARCH DESIGN AND METHODS

## Reagents

Evans blue (Sigma-Aldrich, St. Louis, MO) was dissolved in normal saline (30 mg/ml); Streptozotocin (STZ, Sigma-Aldrich, St. Louis, MO) was dissolved into 0.05M citrate buffer (pH 4.5). Erythropoietin (r-Hu-EPO, Sigma-Aldrich, St. Louis, MO) was diluted in normal saline (0.1 $\mu$g/$\mu$l); in situ Cell Death Detection Kit and EPO ELISA Kit were purchased from Roche Applied Science (Mannheim, Germany).

## Experimental animals and intravitreal EPO treatment

Male Sprague-Dawley rats of approximate 150 g body weight (Slaccas, SIBS, Shanghai, China) were utilized. The animals were treated in accordance with the ARVO Resolution on the Care and Use of Laboratory Animals. For production of diabetes, a single streptozotocin (STZ) intra-peritoneal injection (60 mg/kg body weight in citric buffer) was performed after the rats had been fasted for 24 hours. The control rats received an equal volume of citric buffer, and were monitored as the diabetic rats. All animals were maintained on a 12-h alternating light/dark cycle, and allowed to eat and drink ad libitum, except for the day of fasting before the STZ injection. Animals receiving STZ were declared diabetic when their blood glucose exceeded 250 mg/dL for three consecutive days on daily measurement, and then at weekly intervals. The rats were excluded from the experiment if they failed to develop diabetes. The diabetic rats were randomly divided into two groups: EPO treated and non-treatment groups. Their body weight was recorded twice a week, and 4 units NPH insulin were administered subcutaneously once a week to prevent ketosis. The rats were sacrificed at 1, 2, 4 and 6 weeks after the onset of diabetes. Intravitreal injection of EPO was performed within 2 hours after the onset of diabetes. After systemic anesthesia with intravenous 2% pentobarbital sodium and topical corneal anesthesia with 2% Lidocaine, EPO (r-Hu-EPO in normal saline) was injected into the vitreous with a micro-syringe (Hamilton, Switzerland) and a 30 gauge, ½ inch needle (Becton Dickinson & Co. Rutherford, N.J.), using a temporal approach, 2 mm posterior to the limbus to avoid damaging the lens. EPO, ranging from 0.05ng to 200ng per eye, was dissolved in an equal volume of 2 $\mu$l Sham injections (2 $\mu$l normal saline) were performed to both non-diabetic control rats as well as the diabetic rats in untreated group. The rats recovered spontaneously from the anesthesia and then were sent back to the animal room with food and water ad libitum.

## Examination of Blood-Retinal Barrier Permeability

The Blood-Retinal Barrier (BRB) permeability was evaluated according to published methods. Briefly, the rats were anesthetized with intraperitoneal 2% pentobarbital sodium (50mg/kg body weight). Additional anesthesia was provided throughout the procedures as needed. The left iliac artery and vein were cannulated with a catheter (BD Insyte), that was filled with heparinized saline (400 units heparin/mL saline). The Evans blue solution (30mg/ml) was injected through the left iliac vein over 10 seconds. Two minutes after the Evans blue administration, 0.1 ml blood was drawn from the left iliac artery. An equal volume of the blood was then drawn at 15-minute intervals up to 2 hours after the injection to obtain the time-averaged Evans blue plasma concentration. After the dye had circulated for 2 hours, the chest cavity was opened, and rats were perfused via left ventricle with 1% paraformaldehyde in 0.05M citric acid (pH 3.5) at 37°C. The perfusion lasted 2 minutes at a physiological pressure of 120 mmHg. Immediately after the perfusion, both eyes were enucleated and the anterior segment was removed. The retina was then carefully separated under an operating microscope and dried for approximately 2 hours in a Speed-Vac (37°C). The two eyes from the same animal were pooled together. After determining the dry weight, the retinas were incubated in 300 $\mu$l formamide (Sangon,

Shanghai, China) for 18 hours at 70°C. The extract was centrifuged through 30,000 NMWL centrifuge filter (Microcon, Millipore) for 45 minutes at 3,000 g, 4°C. The volume of the filtrate was measure after centrifugation. The blood samples were centrifuged at 10,000 g for several seconds and diluted to 1:5,000. Sixty microliters of the samples were used for triplicate measurements with a spectrophotometer (Beckman DU800) at a 5-second interval. A background-subtracted absorbance was determined by measuring each sample at both 620nm, the maximum absorbance for Evans blue, and 740nm, the wavelength of minimum absorbance. The concentration of the dye in the blood samples and in the retinal extracts was calculated from a standard curve of Evans blue in formamide. The result of Evans blue permeation into the retina (EBP), as a function of BRB permeability, was calculated using the following equation, and expressed in ($\mu$l plasma X g retinal dry wt$^{-1}$·hr$^{-1}$).

$$\frac{\text{Evans blue } (\mu/\text{pooled retinal dry weight (g)}}{\text{Time - averaged Evans blue concentration } [(\mu]}$$

## Sample preparation for morphologic studies

Rats were sacrificed with deep anesthesia followed by cervical dislocation. A cutting mark was made at 3 and 9 clock of the limbus for orientation. For cryostat section sample preparation, the eyes were enucleated and fixed in PBS-buffered 4% paraformaldehyde for 24 hours and then were opened along the ora serrata and the posterior eyecups dehydrated through a gradient concentration of sucrose in 0.01 mol/L phosphate buffer from 10% to 30%. Following the dehydration, the eyecups were embedded in optimal cutting temperature (OCT) compound (Tissue Tek; Sakura Japan) for sectioning.

The eyes were oriented by the optic nerve head and pre-enucleated cuts, and sections of the retina were prepared along the dorsal-ventral plane of the eye. Serial sections that passed through the optic nerve head were analyzed. For the retinal thickness, cell counting and TUNEL staining, 10-$\mu$m-thick sections were used.

## Measurement of the changes in retinal thickness and cell counts

The cryosectioned retinas were stained with hematoxylin and eosin. The thickness of the different retinal layers was measured under 200X magnification, including: (1) outer limiting membrane to inner limiting membrane (OLM-ILM); (2) outer limiting membrane to ganglion cell layer (OLM-GCL); (3) outer nuclear layer and outer plexiform layer (ONL-OPL); (4) inner nuclear layer (INL); and (5) inner plexiform layer (IPL). Two measurements were taken on each section, at the two reference lines, which were 1 mm away from the optic nerve on both superior and inferior sides. Data from 5 to 6 rats (both eyecups) were averaged for comparison, to avoid any potential anatomic variations in different topographic regions. The cells numbers in ONL, INL and GCL were counted in the same region under the magnifications (1,000X). All the cells within a fixed 25-$\mu$m column, centered with the 1 mm reference lines, were counted (by counting the nucleuses). The cell density was then expressed as the cell count/mm width of retina in different layers.

## In situ detection of cell death in retina by TUNEL assay

The TUNEL assay was performed using In Situ Cell Death Detection Kit, following the manufacture's instructions with minor modifications. The cryosectioned sample that had been stored in 4% paraformaldehyde in 0.1mol/L phosphate buffer, were equilibrated to room temperature for 20 minutes, and then washed in PBS for 30 minutes. The washed sections were incubated in freshly prepared 0.1% Triton x-100 in 0.1% sodium citrate for 2 minutes at 4°C. The sections were rinsed twice with PBS and dried. Following the addition of 10$\mu$l TUNEL reaction mixture, the sections were incubated in a dark, humidified atmosphere for 60 minutes at 37°C. Positive controls were retinal sections that had been treated with Grade I DNase-I, for 10 minutes at room temperature prior to the labeling procedure. Negative controls were the retinal sections treated with 10$\mu$l label solution but incubated in the absence of terminal transferase. The sections, rinsed 3 times with PBS after incubation, were directly analyzed under an epifluorescence microscope (Nikon, Japan) and evaluated with an excitation wavelength in the range of 450-490nm.

## Electron microscopy

The rats were sacrificed with deep anesthesia (intraperitoneally 2% pentobarbital sodium, 50mg/kg body weight). Following the systemic perfusion, both eyes were enucleated, dissected along the equators, and immersed in 2.5% glutaraldehyde in 0.1 mol/L phosphate buffer (pH 7.4) for 1 day. The eye were then fixed in 1% osmium tetroxide, dehydrated through a graded ethanol series and then embedded in EPOn 618 (TAAB Laboratories Equipment, Berks, UK). Ultra-thin sections of the posterior region of the retina were stained with lead citrate and uranyl acetate, and then examined under an electron microscope at 100 kV (GM-120; PHLIP, Holland). All types of cells in the retinal samples were examined by the morphological characteristics.

**Estimation of the half-life elimination of EPO in the eyes**

After intravitreal injection of EPO, the rats were sacrificed as the time points indicated in the legend of Figure 9. After enucleation of eyes, the external ocular tissue was trimmed away. The outer surface of the eyes was further dried with filter paper, and then the anterior segment was removed. The remaining posterior portion of each eye was vertically placed in an Eppendorf tube. After centrifugation at 700g for 15 minutes at room temperature, 50 $\mu$l of vitreous fluid pooled from 4 eyes were collected for EPO quantification using EPO Elisa Kit following the instructions provided by the manufacturer.

**Statistics**

Data are expressed as mean $\pm$ S.E. The statistical analyses were carried out using Student's $t$-test. A $P$-value of 0.05 or less was considered statistically significant.

**RESULTS**

**Establishment of diabetic model in rats with and without EPO treatment**

After a single intra-peritoneal injection of STZ over a 1- to 4- week period, serum glucose levels in the diabetic group of rats increased to 4 to 6 folds compared with the control condition (FIG. 1A). The body weight of the diabetic rats decreased 7.7% after 1 week and then remained relatively constant between 2- to 4- weeks, while the body weight of the non-diabetic control animals had increased 74.3% at 4 weeks (FIG. 1B). At 4 weeks after the onset of diabetes, the body weight of the diabetic rats was 70% of that in age-matched non-diabetic control group. EPO injected intravitreally (50 ng/eye) had no effect on body weight of either the normal group or diabetic group of animals.

**BRB breakdown in the early stage of diabetes and the protection by intraocular EPO**

BRB permeability was assessed with Evens blue quantification because this technique has been demonstrated to be reliable in rats in early diabetes. To exclude the possible BRB permeability increase due to the increased unbound Evans blue in the acidic condition, e.g, under the condition of diabetic ketoacidosis, the serum pH was monitored in diabetic rats treated with insulin.

Table 1 shows the blood pH measurements of STZ-diabetic rats treated with and without insulin as well as with and without EPO. (Control, the rats received sham injection (normal saline intravitreal injection); EPO+Insulin, the diabetic rats received intravitreal injection of EPO (50ng/eye) and treated with subcutaneous injection of insulin (4 units/week); EPO-Insulin, the diabetic rats received EPO intravitreal injection but no insulin treatment.)

**TABLE 1**

| Groups | N | pH (means$\pm$S.E.) | p value |
|---|---|---|---|
| Normal control | 5 | 7.444$\pm$0.17 | 0.446 |
| EPO + Insulin | 6 | 7.409$\pm$0.013 | 0.053 |
| EPO - Insulin | 6 | 7.430$\pm$0.011 | 0.125 |

As shown in Table 1 and FIG. 2, the insulin therapy did not significantly alter serum pH and did not interfere with the function of EPO on vascular permeability at 2-weeks after the onset of diabetes. Therefore, the constant serum pH in diabetic group ruled out the possible influence of pH on the quantification of Evans blue.

To monitor vascular permeability in STZ-diabetic rats, Evans blue-albumin permeation (EBP) was measured from 1 to 4 weeks after diabetes onset. FIG. 3 shows that EBP of STZ-diabetic rats increased 56.8% after 1 week (n=8, p<0.05) in comparison with the non-diabetic rats (n=10), and reached a peak after 2 weeks (62.9% increase, n=7, p<0.05). The EBP remained at elevated levels between 2- to 4-weeks (Fig. 3, n=8 p<0.05). In FIG. 3, in the first two weeks, all three dosages of EPO (5, 20 and 50ng/eye) exhibited similar, effective protection of BRB. After three weeks, the lowest dose, 5ng/eye, of EPO treatment demonstrated loss of its protective function on BRB (n=4; p > 0.05). At four weeks after the onset of diabetes, only the higher dose of EPO, 50ng/eye still showed significant protection to BRB against the diabetic insult (n=8; p < 0.05). When greater dosages of EPO were used (up to 200ng/eye), a dose-dependency of EBP was observed (Fig 4 at 2 weeks after onset of diabetes). At the highest concentration (200 ng/eye), the protective role of EPO on the integrity of BRB was less effective, but still maintained the EBP significantly lower than the diabetic group (p<0.05). The optimal effect of EPO was obtained at 50ng/eye level (FIG 4).

The data indicate that a single intravitreal injection of EPO at a proper dosage can preserve the BRB function for at least 4 weeks after the onset of diabetes. In FIG. 3, surprisingly the EBP in EPO-treated diabetic groups was significantly lower than in the group of non-diabetic control (sham injection group) at the 2-week interval (n=8; p<0.05). It is possible that the altered EBP by sham intravitreal injection can also be prevented by EPO treatment.

**Protection of diabetic retina by EPO from reduction in thickness and loss of neurons in different layers**

Morphometric examination of hematoxylin and eosin-stained retinal cryostat sections demonstrated significantly reduced total retinal thickness in diabetic rats after 1 week of diabetes onset in comparison with non-diabetic control rats (FIG. 5). Primarily the reduction in thickness occurred in the ONL in diabetic rats up to 4 weeks (n= 72; p<0.001). By 4 weeks after diabetes induction, there were significantly fewer cells in ONL (84.61%, n=52; p<0.001), and marginal reductions in cell number in INL (98.21%, n=54; p>0.05), when the retinal sections of diabetic and non-diabetic control eyes were compared (Fig 6). For diabetic rats that were treated with intravitreal injections of EPO (50ng/eye), the total retinal thickness and cell numbers in ONL were not different from the non-diabetic control for the entire 4 weeks after onset of diabetes (FIG. 5 & FIG. 6.). There was no significant difference in thickness and cell number of non-diabetic control animals in the time course observed (n = 52, p>0.05).

**Protection of retinal neurons from cell death by EPO in diabetes**

To characterize the cell death in GCL, INL and ONL of diabetic and non-diabetic rats, TUNEL analysis was used, although this technique also detects DNA fragments that may result from necrosis (26). In the diabetic untreated eyes, TUNEL-positive cells significantly increased in ONL from 1 week and reached the peak at 4 weeks after diabetes induction (FIG. 7). In combination with the electron microscopic study, minimal necrosis was noticed in the ONL of the diabetic eyes, indicating TUNEL-positive staining is a marker of apoptosis. The number of TUNEL-positive cells observed in GCL and INL of the diabetic rats were strikingly less than that in ONL (FIG. 7). After intravitreal injection of EPO (50 ng/eye), essentially no TUNEL-positive cells were detected in GCL, INL and ONL from 1- to 3- weeks after onset of diabetes. However, some TUNEL-positive cells were noted in ONL at 4 weeks (FIG. 7) and 6 weeks (data not shown) of the EPO-treated diabetic eyes. The number of TUNEL-positive cells was significantly lower than that in untreated eyes of diabetic rats, but greater than in the non-diabetic controls (Fig. 7).

**EM evidence of a protective effect of EPO on retinal vascular cells and neurons from diabetic insult**

The representative ultrastructural changes of the rat retinas were illustrated in FIG. 8. The cell and tissue alterations in different retinal layers could be detected at 7-10 days after onset of diabetes in comparison with non-diabetic retina. Prominent changes of microvascular endothelial cells (MEC) were discerned including apoptotic nuclear condensations accompanied by para-vascular edema and exudates (FIG. 8. en-D). In the retinal pigment epithelium (RPE), disruption of Bruch's membrane (BM), and the accumulation of collagen were observed in diabetic eyes, resulting the widened space between the BM and RPE (FIG. 8. rpe-D). The structure of the rod and cone layers was severely damaged with widened intradisc spaces left by the dissolved segments (Fig. 8. r/c-D). The striking change in the outer nuclear layer (ONL) was the appearance of pyknosis, which correlated with the data of cell loss and increased TUNEL-positive cells in ONL (vide supra) (FIG. 8. onl-D). At the same points in time, the diabetic rats treated with EPO treatment showed either no change or only minor changes (FIG. 8. en-E, rpe-E, r/c-E and onl-E), in comparison with the non-diabetic control group (FIG. 8. en-C, rpe-C, r/c-C and onl-C).

**Half-life of vitrous EPO after intravitreal injection**

In non-diabetic rats, the elimination half-life of EPO after intravitreal injection ranged from 24 to 36 hours. After the maximum concentration reached, the EPO levels decreased rapidly. At 72 hours after injection, the EPO concentration returned to the baseline (Fig. 9). The pharmacokinetic profile of EPO in the vitreous following intravitreal delivery is eliminated with first order kinetics.

**DISCUSSION**

Diabetic retinopathy, by its classic definition, is recognized to be a microangiopathy occurring with diabetes. The microangiopathy is characterized by BRB breakdown, capillary basement membrane thickening, loss of pericytes and the development of acellular, occluded capillaries. The STZ-induced diabetic rat model displays similar morphological and functional changes in the retinal vasculature as that observed in the early stage of human diabetic retinopathy. Increased apoptosis of microvascular pericytes and endothelial cells has been well established as an important part of the vascular damage in diabetic retinas of both humans and rats. The apoptotic processes in retinal vascular cells are not just late event of diabetes. It has been reported that in STZ-diabetic rat model retinal microvascular endothelial cell apoptosis significantly increases after 9- to 14- days compared with the non-diabetic retina.

The present study investigated the alterations of BRB function and cellular injury of both the inner and outer BRB from 1 day to 4 weeks after diabetes onset. This study demonstrated the breakdown of the BRB as early as 4 days after the onset of diabetes (data not shown). BRB breakdown at such early stage in diabetic rats also has been reported by others. Vascular endothelial cell apoptosis was demonstrated by EM in this study as early as 7-10 days after diabetes onset (FIG. 8. en-D). RPE cells *per se* did not show significant injury at this time, but ruptures in the adjacent Bruch's membrane, and the widened space between RPE and Bruch's membrane, were observed (FIG. 8 rpe-D). However, a functional defect in permeability of the RPE has been detected at 4 weeks by others in STZ diabetic rats.

Increasing evidence shows that retinal neuronal pathology progresses along with alterations of other cells (such as the glia) very early in diabetes. Some authors have suggested that diabetic neuronal or glial pathology may occur prior to microangiopathy. In the present study, apoptotic neurons were detected by TUNEL staining mainly in outer nuclear layer (ONL) with small numbers in INL and GCL (FIG. 7) as early as 1 week after the onset of diabetes. The increased TUNEL staining is presumably attributed to apoptosis, and unlikely due to ischemic necrosis because at this stage no capillary occlusion or other signs of necrosis were observed. This apoptotic alteration of neurons, particularly photoreceptors, was also corroborated by electron microscopy (FIG. 8). Our findings suggest that both vascular cells and neural cells die by apoptosis in early diabetes. The parallel time course of microangiopathy and neuronopathy (different from diabetic peripheral neuropathy) indicates that a pervasive diabetic insult is the trigger of apoptotic process during very early stages. Therefore, protecting both vascular cells, glial, and neurons from apoptotic insult should be a high priority and implemented as early as possible.

In the experimental STZ-diabetic rat model, the intravitreal injection of EPO demonstrated a dose-dependent inhibition of the breakdown of the BRB that followed a U-shaped response (Fig 4), characteristic of cytokines. Specifically, the present dose-dependent curve showed less efficacy at the high dose of 200 ng/eye than a maximal response noted at 50ng/eye. It is interesting to note that in EPO-treated groups leakage levels determined by Evans blue that were even lower than the control (sham injection) at the second week (FIG 3). This suggests that the trauma due to the injection alone leads to an increase in Evans blue leakage. This trauma induced leakage was spontaneously recovered in the control eyes after a period of 2 weeks. At this period, the protective effect of EPO against the breakdown of the BRB may be related to the anti-inflammatory effect known of EPO. The molecular mechanism by which EPO protects BRB against its elevated permeability in diabetes is currently under investigation.

The present data provide the morphological evidence of neuron injury, which can be used to explain the visual dysfunction in early diabetic rats as reported previously. In order to explore whether EPO can prevent or delay the neuronal apoptosis, intravitreal injection of EPO was performed at the day of diabetic onset in the present study. Apoptotic neurons in ONL were essentially undetectable in EPO treated diabetic eyes up to 4 weeks (FIG. 7). The apoptotic figures in 6-week diabetic rats treated with EPO were significantly reduced but detectable (data not shown). In addition, the thickness of the ONL in EPO treatment groups up to 4 weeks was essentially unchanged (FIG. 5). A slightly reduced ONL thickness was observed in the 6-week diabetes (data not shown). These findings suggest that the therapeutic efficacy of a single intravitreal injection of EPO may persist for at least 1 month, even though the vitreous EPO concentration returned to baseline within 3 days (FIG. 9).

The present study is the first to show that an intravitreal injection of EPO protects the function and integrity of BRB, and prevents the vascular/neuronal/glial/RPE cell apoptosis in the early diabetic retina. In the study of other tissues, evidences exist that EPO mediates protective functions by maintaining normal vascular autoregulation through increased endothelial nitric oxide synthase. It also has been reported that EPO maintains vascular integrity by antagonizing inflammation in other experimental models. It has been proposed that the apoptotic process in EPO-receptor expressing cells could be aborted by the rapid binding of EPO. In an in vitro study, apoptosis of neurons was stopped by as brief an exposure to EPO as 5 min. In the spinal cord injury model, a single dose of EPO immediately following injury produced a recovery that was superior to the animals treated with multiple doses of EPO. Nevertheless, the mechanism by which a single intravitreal injection of EPO produces an effect for 4 weeks in diabetic rat eyes is currently unknown since the elimination half-life from the vitreous of EPO was observed to be 24 to 36 hours (FIG. 9) with a return to baseline by 72 hours. The mechanism of the cytoprotective effect of EPO appears different from that of the erythropoietic function which requires sustained levels of EPO to stimulate erythroid precursors in the bone marrow. Based on the present data, it appears that a significant protective function of EPO can be provided through episodic delivery into the vitreous and may provide as a therapeutic approach to diabetic retinopathy in its earliest stages.

## EXAMPLE 2

### Intervention of Diabetic Retinopathy in STZ rats with EPO

### Methods

Diabetes was induced in SD rats by STZ (i.p.) as in the previous experiments. A single intravitreal injection of EPO (about 8mU to about 160mU/eye) was given either at 1 (group 1) or 4 weeks (group 2) after diabetes onset. The eyes were examined at 2 weeks after injection in Group 1 or 2 and 5 weeks after injection in Group 2. The blood-retinal barrier (BRB) was monitored by Evans blue technology. Apoptosis was detected by TUNEL. The retinal thickness and cell counts in different layers were evaluated with light microscopy. EM was used to scrutinize vascular and neuronal injury.

### Results

EPO treatment (100ng/eye) significantly rescued Blood-Retinal Barrier (BRB) function in STZ rats treated with EPO, which was given one week after the onset of diabetes, even though the albumin permeation into the retina did not reduce to the control level (Figure 10). Intravitreal EPO treatment (8-160mU/eye) significantly rescued the apoptosis of retinal neurons in STZ treated rats (Figure 11). EPO treatment significantly prevented/rescued the retinal neurons from death caused by STZ. The retina became thinner in STZ rats and the change was evidently improved by intravitreal injection of EPO at different times after the onset of diabetes (Figure 12A). STZ rats also significantly lost retinal neurons in the out nuclear layer (ONL) and the cell counts in ONL in STZ rats treated with EPO at different times after the onset of diabetes were similar to those in the control rats (Figure 12B). EPO treatment significantly prevented/rescued the retinal neurons from death caused by STZ, EM evidence. The ultra microstructure of retina in STZ rats was damaged evidently. However, the retinal cells basically showed normal structures (Figure 13).

### Conclusion

Apoptosis of retinal neurons is a critical constituent in early diabetic retinopathy (EDR). Intravitreal injection of EPO stopped or even reversed the changes caused by STZ in retina, as showed by its protection to BRB function and retinal neurons from death. Therefore, EPO appears to be a novel approach for treatment of EDR.

## EXAMPLE 3

### Toxicity study on EPO intravitreal injection in rabbits

### Purpose:

To evaluate the retinal toxicity of varying doses of EPO when injected intravitreally in rabbits. (EPO was approved by the US FDA for the treatment of anemia.)

### Materials and Methods:

New Zealand albino rabbits were used for this study and divided into four groups. Four doses of EPO were prepared: 10U/eye, 100U/eye, 1,000U/eye for single injection and 0.6U/eye (the dosage for normal treatment) for monthly repeated injections. Each concentration was injected intravitreally into the left eye of the rabbits (n=6 in each group), shame injection was performed with equivalent volume of normal saline into the contra lateral eyes. Slit-lamp and funduscopic examinations were performed and the animals were observed for at least 6 months for signs of infection, inflammation, cataract or any other toxic responses. Fluorescent fundusphotography (FFA) was performed to detect the leakage of blood vessels.

Baseline flash electroretinogram (ERG) was performed to examine the functions of photorecepters and some other retinal neurons before the EPO injection, and at different time points after EPO injection (1 day, 3 days; 1, 2 and 4 weeks, as well as 6 months. VEP was performed in 1, 2 and 3 months. Hematology study included: cell counts (before ITV and in 1, 2 and 3 months after ITV), EPO concentration in the blood immediately after ITV, Anti-EPO antibody in the blood or vitreous (1 or 3 months after ITV).). Allergic reaction was also monitored. The enucleated eyes were prepared for histological evaluation of retinal toxicity. The rabbits were killed at 1 week, 1 month, 2 months and 3 months after ITV

**Results:**

Routein slit-lamp examination and fundus examination showed no abnormal change in cornea, anterior chamber, iris, lens, vitrouse and retina, up to 2 months after the intravitreal injection of different dosages of EPO. No sign for anterior segment inflammation, cataract or retinopathy was found (Figure 14).

Fluorescein Angiography (FFA) also showed no leakage in the fundus after EPO intravitreal injections (Figure 15).

Electroretinography (ERG) examination is one of the few methods to evaluate the functions of photorecepters and other related retinal neurons. In the president study, ERG was employed to detect the early changes in retinal functions. It showed no defect in ERG response to the flash stimulation. No significant difference between these groups was detected (Figure 16).

Rabbits retinas were examined under light microscopy for its thickness and cell counts. No significant difference in either parameters was observed (Figure 17).

The histological observation for EPO toxicity in retina of rabbits showed no abnormal structural change and no inflammation and other obvious abnormality (Figure 18).

**Conclusion :**

Intravitreal injection of EPO does not cause inflammation, cataract or other eye fundus diseases in the rabbit eyes; maintain the BRB function; does not disturb the normal retinal electrophysiological activities and does not cause the structure changes in the eyes. The data showed here indicate that intravitreal injection of EPO (0.6U/eye to 1,000U/eye) is safe in rabbit eye, for at least two months.

**EXAMPLE 4**

**Comparison of different solutions to resolve EPO on protection to the retina of STZ rats**

Diabetes was induced in rats by a single STZ intra-peritoneal injection (60 mg/kg BW in citric buffer) as described before. The diabetic rats were randomly divided into two groups: EPO treated and non-treated groups. EPO (8mU/eye) was dissolved in four different kinds of solutions to test the effects of the solution to the therapeutic functions. The four different solvents are normal saline (NS), phosphate-buffered saline (PBS), balanced saline solution (BSS) and sodium lactate ringer's injection (Ringer). EPO was dissolved in an equal volume of 2 μl. Sham injections (2 μl normal saline) were performed to both non-diabetic control rats as well as the untreated diabetic rats. The rats were killed to do Evens Blue Permeation (EBP) experiment at 2 weeks after diabetes onset. The method for EBP experiment is the same as the methods described previously.

**Results:**

EPO dissolved in all the four different solutions and all EPO (in any solution) showed protective effects BRB, as a parameter we selected to test the effects of the solution (Figure 19).

**Claims**

1. Use of erythropoietin for the manufacture of a medicament for the treatment of a retinal disorder associated with breakdown of blood-retinal-barrier or a retinal disorder associated with apoptosis of cells of retina, wherein the retinal disorder is diabetic retinopathy.

2. The use of claim 1, wherein the medicament comprises a composition for invitreal injection.

3. The use of claim 1, wherein the cells of retina are photoreceptor cells of the outer nuclear layer, neurons of the inner nuclear layer, retinal pigment epithelial cells, and/or choriocapillaris endothelial cells.

4. The use of claim 1, wherein the medicament comprises an effective amount of erythropoietin of about 1.33 μg to about 60 μg per eye.

5. The use of claim 1, wherein the medicament comprises an effective amount of erythropoietin of about 0.23 to about 9.2 units per eye.

**6.** The use of claim 1, wherein the erythropoietin comprises human recombinant erythropoietin.

**7.** A composition comprising erythropoietin for use in the treatment of a retinal disorder associated with breakdown of blood-retinal-barrier or apoptosis of cells of retina, wherein the retinal disorder is diabetic retinopathy.

**8.** The composition for use according to claim 7, wherein the cells of retina are photoreceptor cells of the outer nuclear layer, neurons of the inner nuclear layer, retinal pigment epithelial cells, and/or choriocapillaris endothelial cells.

**9.** The composition for use according to claim 7, wherein the composition comprises an effective amount of erythropoietin of about 1.33 $\mu$g to about 60 $\mu$g per eye.

**10.** The composition for use according to claim 7, wherein the composition comprises an effective amount of erythropoietin of about 0.23 to about 9.2 units per eye.

**11.** The composition for use according to claim 7, wherein the composition comprises human recombinant erythropoietin.


**Patentansprüche**

**1.** Verwendung von Erythropoietin zur Herstellung eines Medikaments zur Behandlung einer Netzhauterkrankung, die mit einer Zerstörung der Blut-Netzhaut-Schranke verbunden ist, oder einer Netzhauterkrankung, die mit Apoptose von Netzhautzellen verbunden ist,
wobei es sich bei der Netzhauterkrankung um diabetische Retinopathie handelt.

**2.** Verwendung nach Anspruch 1, wobei das Medikament eine Zusammensetzung zur Injektion in den Glaskörper umfasst.

**3.** Verwendung nach Anspruch 1, wobei es sich bei den Netzhautzellen um Fotorezeptorzellen der äußeren Körnerschicht, Neuronen der inneren Körnerschicht, Netzhautpigment-Epithelzellen und/oder Endothelzellen der Lamina choroidocapillaris handelt.

**4.** Verwendung nach Anspruch 1, wobei das Medikament eine effektive Menge Erythropoietin von ungefähr 1,33 $\mu$g bis ungefähr 60 $\mu$g pro Auge umfasst.

**5.** Verwendung nach Anspruch 1, wobei das Medikament eine effektive Menge Erythropoietin von ungefähr 0,23 bis ungefähr 9,2 Einheiten pro Auge umfasst.

**6.** Verwendung nach Anspruch 1, wobei das Erythropoietin humanes rekombinantes Erythropoietin umfasst.

**7.** Zusammensetzung, umfassend Erythropoietin zur Verwendung bei der Behandlung einer Netzhauterkrankung, die mit einer Zerstörung der Blut-Netzhaut-Schranke oder mit Apoptose von Netzhautzellen verbunden ist,
wobei es sich bei der Netzhauterkrankung um diabetische Retinopathie handelt.

**8.** Zusammensetzung zur Verwendung nach Anspruch 7, wobei es sich bei den Netzhautzellen um Fotorezeptorzellen der äußeren Körnerschicht, Neuronen der inneren Körnerschicht, Netzhautpigment-Epithelzellen und/oder Endothelzellen der Lamina choroidocapillaris handelt.

**9.** Zusammensetzung zur Verwendung nach Anspruch 7, wobei die Zusammensetzung eine effektive Menge Erythropoietin von ungefähr 1,33 $\mu$g bis ungefähr 60 $\mu$g pro Auge umfasst.

**10.** Zusammensetzung zur Verwendung nach Anspruch 7, wobei die Zusammensetzung eine effektive Menge Erythropoietin von ungefähr 0,23 bis ungefähr 9,2 Einheiten pro Auge umfasst.

**11.** Zusammensetzung zur Verwendung nach Anspruch 7, wobei die Zusammensetzung humanes rekombinantes Erythropoietin umfasst.

**Revendications**

1. Utilisation d'érythropoïétine pour la fabrication d'un médicament destiné au traitement d'un trouble rétinien associé à une rupture d'une barrière hémato-rétinienne ou d'un trouble rétinien associé à l'apoptose de cellules de la rétine, dans laquelle le trouble rétinien est une rétinopathie diabétique.

2. Utilisation selon la revendication 1, dans laquelle le médicament comprend une composition pour injection intravitréenne.

3. Utilisation selon la revendication 1, dans laquelle les cellules de la rétine sont des cellules photoréceptrices de la couche nucléaire externe, des neurones de la couche nucléaire interne, des cellules épithéliales pigmentaires rétiniennes, et/ou des cellules endothéliales choriocapillaires.

4. Utilisation selon la revendication 1, dans laquelle le médicament comprend une quantité efficace d'érythropoïétine d'environ 1,33 μg à environ 60 μg par oeil.

5. Utilisation selon la revendication 1, dans laquelle le médicament comprend une quantité efficace d'érythropoïétine d'environ 0,23 à environ 9,2 unités par oeil.

6. Utilisation selon la revendication 1, dans laquelle l'érythropoïétine comprend de l'érythropoïétine humaine recombinante.

7. Composition comprenant de l'érythropoïétine pour son utilisation dans le traitement d'un trouble rétinien associé à une rupture d'une barrière hémato-rétinienne ou à l'apoptose de cellules de la rétine, dans laquelle le trouble rétinien est une rétinopathie diabétique.

8. Composition pour son utilisation selon la revendication 7, dans laquelle les cellules de la rétine sont des cellules photoréceptrices de la couche nucléaire externe, des neurones de la couche nucléaire interne, des cellules épithéliales pigmentaires rétiniennes, et/ou des cellules endothéliales choriocapillaires.

9. Composition pour son utilisation selon la revendication 7, dans laquelle la composition comprend une quantité efficace d'érythropoïétine d'environ 1,33 μg à environ 60 μg par oeil.

10. Composition pour son utilisation selon la revendication 7, dans laquelle la composition comprend une quantité efficace d'érythropoïétine d'environ 0,23 à environ 9,2 unités par oeil.

11. Composition pour son utilisation selon la revendication 7, dans laquelle la composition comprend de l'érythropoïétine humaine recombinante.

FIG. 1

FIG. 2

FIG. 3

FIG. 4

FIG. 5

FIG. 6

FIG. 7

FIG. 8

FIG. 9

FIG. 10

FIG. 11

FIG. 12

FIG. 13

FIG. 14

FIG. 15

FIG. 16

FIG. 17

FIG. 18

FIG. 19

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 640619 A
- WO 9966054 A
- WO 9938890 A
- US 5688679 A
- WO 9911781 A
- WO 9805363 A
- US 5643575 A
- WO 9905268 A
- WO 9412650 A
- US 6340742 B, Burg
- US 6310078 B, Connolly
- US 6251090 B
- US 6299603 B
- US 20050250737 A
- US 20050208103 A
- US 20050074497 A
- US 20050064010 A
- US 4954437 A
- US 4703008 A
- WO 8502610 A
- EP 232034 B1
- US 6325998 B

**Non-patent literature cited in the description**

- **FONG et al.** *Diabetes Care,* 01 January 2004, 84-87
- Transscleral drug delivery for posterior segment disease. *Adv Drug Delivery Rev,* vol. 52, 37-48
- **JACOBS et al.** *Nature,* 1985, vol. 313, 806-810
- **LIN et al.** *Proc. Natl. Acad. Sci. USA,* 1985, vol. 82, 7580
- **KOHNER E. M. et al.** *Diabetic Retinopathy Metabolism,* 1975, vol. 25, 1985-1102